# EUROPEAN PATENT APPLICATION

(11) **EP 2 058 004 A1**
(43) Date of publication of application: **13.05.2009**
(21) Application number: 09152737.4
(22) Date of filing: 16.03.2006
(51) Int. Cl.: A61K 39/21, C12N 7/00, C12P 21/02, C12P 1/02, C07K 14/16

(54) **Mannose immunogens for HIV-1**

(30) Priority: 16.03.2005 US 661933 P; 26.10.2005 US 730019 P
(62) Divisional of application: 06748437.8
(71) Applicant: University of Oxford, South Parks Road Oxford OX1 3QU (GB)
(72) Inventor: Dwek, Raymond, Oxford Oxfordshire OX2 9AU (GB); Rudd, Pauline M., Abingdon OX14 2PA (GB); Ritchie, Gayle, Slough SL1 6LS (GB); Scanlan, Christopher, Oxford OX2 7BW (GB); Crispin, M.D. Max, Oxford OX3 9BG (GB)
(74) Representative: Warcoin, Jacques

(57) **Abstract**

Methods of producing a carbohydrate HIV vaccine or immunogenic composition are provided. One method comprises expressing a glycoprotein with a modified glycosylation, which facilitates binding of the glycoprotein to the 2G12 antibody. Another method comprises iteratively selecting cells with a high affinity for the 2G12 antibody.

## Description

The present application relates generally to carbohydrate engineering and, in particular, to carbohydrate human immunodeficient virus (HIV) vaccines and/or immunogenic compositions and methods of making such vaccines and compositions.

### BACKGROUND

Anti-carbohydrate recognition represents a major component of both adaptive and innate immunity. However, only in a limited number of cases has the protective nature of antibodies to surface carbohydrates been exploited in a vaccine design. The antigenic role of glycosylation is of particular significance in the case of human immunodeficiency virus type 1 (HIV-1). The surface of HIV-1 is covered by large, flexible and poorly immunogenic N-linked carbohydrates that form an 'evolving glycan shield' that promotes humoral immune evasion (see, e.g., X. Wei et. al. "Antibody neutralization and escape by HIV-1", Nature, 422(6929), pp. 307-312, 2003, incorporated hereby by reference in its entirety). Three major explanations for the poor immunogenicity of HIV glycans have been proposed. Firstly, the glycans attached to HIV are synthesized by the host cell and are, therefore, immunologically 'self'. Secondly, the binding of a protein to a carbohydrate is generally weak and , thus, limiting the potential for high affinity anti-carbohydrate antibodies. Finally, multiple different glycoforms can be attached to any given *N*-linked attachment site, thus, producing a highly heterogeneous mix of potential antigens. A wide range of complex, oligomannose and hybrid type glycans are all present on HIV, with the oligomannose glycans tightly clustered on the exposed outer domain of gp120. However, antibodies to HIV carbohydrates are not normally observed during infection.

The HIV-1 gp120 molecule is extensively *N-*glycosylated with approximately half the molecular weight of this glycoprotein contributed by covalently attached *N-*glycans. The crystal structure of the gp120 core with *N-*glycans modeled onto the glycoprotein surface identifies one face of the gp120 molecule that contains a cluster of *N-*glycans (see, e.g., P.D. Kwong et. al. "Structure of an HIV gp120 envelope glycoprotein in complex with the CD4 receptor and a neutralizing human antibody", Nature, 393(6686) pp.648-659, 1998, incorporated hereby by reference in its entirety). This face has been denoted the immunologically silent face because only one antibody (2G12) able to recognize this region of the glycoprotein molecule has been identified so far. The *N-*glycosylation of the HIV-1 gp120 molecule is thought to play a major role in immune evasion by preventing antibody accessibility to antigenic protein epitopes that lie underneath the *N-*glycosylation sites. In this instance, the exact structures of the *N-*glycans are of little importance provided they shield the underlying gp120 molecule from antibody recognition. Thus, the gp120 glycan shield can evolve by the introduction of new *N-*glycosylation sites following mutation of the viral genome. This promotes continued evasion of host immunity.

Although antibodies to carbohydrates of HIV are rare, there are many other pathogens, whose carbohydrate moieties elicit a strong antibody response. Indeed, a notable feature of the human humoral anti-carbohydrate reactivity is the widespread existence of anti-mannose antibodies, specific for α1→2 linked mannose oligosaccharides. Unlike 2G12, however, these antibodies do not bind to mannose that is presented within the context of 'self' oligomannose glycans. The probable targets of the natural anti-mannose antibodies are the cell wall mannans present on the lipids and proteins of many commonly occurring yeasts. Immunization with yeast mannans can provide some humoral cross-reactivity with gp120 carbohydrates (see, e.g., W. E. Muller et. al. "Polyclonal antibodies to mannan from yeast also recognize the carbohydrate structure of gp120 of the AIDS virus: an approach to raise neutralizing antibodies to HIV-1 infection in vitro", AIDS. 1990 Feb;4(2), pp. 159-62., incorporated hereby by reference in its entirety; and W. E. Muller et. al. "Antibodies against defined carbohydrate structures of Candida albicans protect H9 cells against infection with human immunodeficiency virus-1 in vitro", J Acquir Immune Defic Syndr. 1991;4(7) pp. 694-703, incorporated hereby by reference in its entirety). However, the titers and affinities observed are not sufficient to warrant use as a prophylactic.

The above notwithstanding, one rare, neutralizing anti-gp120 antibody, 2G12, does bind to a specific carbohydrate epitope on the HIV envelope. The epitope recognized by 2G12 is a highly unusual cluster of mannose residues, present on the outer domain of gp120 (see, e.g., C. N. Scanlan et. al. "The Broadly Neutralizing Anti-Human Immunodeficiency Virus Type 1 Antibody 2G12 Recognizes a Cluster of α1->2 Mannose Residues on the Outer Face of gp120 J. Virol. 76 (2002) 7306-7321, incorporated hereby by reference in its entirety). The primary molecular determinant for 2G12 binding is the α1→2 linked mannose termini of the glycans attached to Asn332 and Asn392 of gp120. This cluster, although consisting of 'self' glycans is arranged in a dense array, highly atypical of mammalian glycosylation, thus, providing a structural basis for 'non-self' discrimination by 2G12. Structural studies of the 2G12 Fab reveal that the two heavy chains of the Fab are interlocked via a previously unobserved domain-exchanged configuration (see, e.g., D. Calarese et. al. "Antibody domain exchange is an immunological solution to carbohydrate cluster recognition", Science, vol. 300, pp. 2065-2071, 2003, incorporated hereby by reference in its entirety). The extended paratope, formed by this domain exchanged Fab, provides a large surface for the high avidity binding of multivalent carbohydrates.

Passive transfer studies of 2G12 indicate that this antibody can protect against viral challenge in animal models of HIV-1. The molecular basis has been elucidated for the broad specificity of 2G12 against a range of HIV-1 primary isolates. Therefore, based on the known structure of the 2G12 epitope, it is highly desirable to develop an immunogen that can be capable of eliciting 2G12-like antibodies and can contribute to sterilizing immunity against HIV-1. However, the design of such an immunogen has to overcome both the structural constraints required for antigenic mimicry of the glycan epitope on gp120 and the immunological constraints inherent to the poorly immunogenic *N-*linked glycans of HIV.

One approach to gp120 immunogen design is to synthetically recreate the antigenic portion of gp120 to which 2G12 binds (see, e.g., H.K Lee et. al. "Reactivity-Based One-Pot Synthesis of Oligomannoses: Defining Antigens Recognized by 2G12, a Broadly Neutralizing Anti-HIV-1 Antibody", Angew. Chem. Int. Ed. Engl, 43(8), pp. 1000-1003, 2004, incorporated hereby by reference in its entirety; H. Li et. al. "Design and synthesis of a template-assembled oligomannose cluster as an epitope mimic for human HIV-neutralizing antibody 2G12", Org. Biomol. Chem., 2 (4), pp. 483 - 488, 2004 incorporated hereby by reference in its entirety; L.-X. Wang, "Binding of High-Mannose-Type Oligosaccharides and Synthetic Oligomannose Clusters to Human Antibody 2G12: Implications for HIV-1 Vaccine Design", Chem. Biol. 11(1), pp. 127-34, 2004, incorporated hereby by reference in its entirety). Presentation of synthetic mannosides in a multivalent format can increase their affinity to 2G12 by almost 100-fold (see, e.g., L.-X. Wang, "Binding of High-Mannose-Type Oligosaccharides and Synthetic Oligomannose Clusters to Human Antibody 2G12: Implications for HIV-1 Vaccine Design", Chem. Biol. 11(1), pp. 127-34, 2004).

Although the synthetic approach to immunogen design is a potentially powerful one, there are significant challenges to the 'rational' design of immunogens. Most fundamentally, the affinity of an antigen for an antibody does not necessarily correlate with the likelihood of that antigen eliciting the evolution of similar antibodies, when used as an immunogen. Thus, it is highly desirable to develop alternative methods of designing an HIV vaccine which will address the inherent limitations of both glycan antigenicity and glycan immunogenicity.

### SUMMARY

The invention provides HIV vaccines and immunogenic compositions, methods of producing such vaccines and compositions and related methods of vaccinating and/or immunogenizing. In accordance with one embodiment, a method of producing an HIV vaccine or immunogenic composition comprises
I) (A) altering a glycosylation pathway of an expression system and
   (B) expressing a glycoprotein in the expression system so that the expressed glycoprotein has a modified glycosylation that increases an affinity of the expressed glycoprotein to the 2G12 antibody or
II) expressing a glycoprotein in an expression system other than a natural expression system of the glycoprotein, wherein the expressed glycoprotein has a modified glycosylation that increases an affinity of the expressed glycoprotein to the 2G12.

Preferably, said altering comprises genetically manipulating the glycosylation that results in a mannosidase deficient cell-line. Alternately, said altering comprises contacting said expression system with an α-mannosidase inhibitor. Preferably, the α-mannosidase inhibitor is Australine, Castanospermine, Deoxynojirimycin, 1,4-dideoxy-1,4-imini-D-mannitol (DIM), Deoxymannojirimycin, 6-deoxy-DIM, Mannostatin A, Swainsonine, D-mannonolactam amidrazone or Propylaminomannoamidine. More preferably, the α-mannosidase inhibitor is Kifunensine. Advantageously, the glycoprotein is a gp120 glycoprotein. Alternately, the glycoprotein is a self glycoprotein. Preferably, the self glycoprotein is a CD48, CD29, CD49a, CD66a, CD80, CD96a, Aminopeptidase or RPTPmu. Alternately, the self-glycoprotein is a soluble glycoprotein construct.

In a further advantageous embodiment, N-glycans of the expressed glycoprotein are predominantly non-glucosylated high mannose glycans. Preferably, said high mannose glycans are selected from the group consisting of Man₉GlcNAc, Man₈GlcNAc, Man₇GlcNAc, Man₆GlcNAc glycans.

Also advantageously, expressing a glycoprotein with a modified glycosylation is carried out in a high-yield mammalian expression system. Preferably, the high-yield mammalian expression system comprises HEK 293T cells, CHO cells or HepG2 cells. In another further advantageous embodiment, the method of the present invention further comprises adding N-linked glycosylation sites on the glycoprotein.

The present invention also relates to HIV vaccine or immunogenic composition produced by a method of the present invention.

According to another embodiment, a method of producing an HIV vaccine or immunogenic composition comprises

performing at least one time an iteration comprising:
(i) selecting from a first pool of cells a subpool of cells, wherein the cells of the subpool have a higher affinity to the 2G12 antibody than the cells of the first pool; and
(ii) replicating the cells of the subpool to produce a second pool of cells; wherein the vaccine or composition comprises the cells of the second pool from a last iteration.

Another aspect of invention is an HIV vaccine or immunogenic composition comprising a glycoprotein, wherein N-glycans of the glycoproteins are predominantly high mannose glycans.

Preferably, said glycoprotein is a gp120 glycoprotein. Alternately, said glycoprotein is a self glycoprotein.

The present invention also relates to a composition comprising a glycoprotein, wherein N-glycans of said glycoprotein are predominantly high mannose glycans for use in the vaccination and/or immunogenization against HIV.

Yet another aspect of the invention is an HIV vaccine or immunogenic composition comprising mannans having a specific complementarity to an epitope of the 2G12 antibody.

Yet the invention also provides an HIV vaccine or immunogenic composition comprising
(i) artificially selected mannans having a specific complementarity to an epitope of the 2G12 antibody; and
(ii) a glycoprotein, wherein N-glycans of said glycoprotein are predominantly high mannose glycans.

Yet according to another embodiment, the invention provides method of vaccinating and/or immunogenizing against HIV, comprising
administering to a subject a composition comprising a glycoprotein, wherein N-glycans of said glycoprotein are predominantly high mannose glycans.

Yet another embodiment is a method of vaccinating and/or immunogenizing against HIV, comprising
administering to a subject a composition comprising artificially selected mannans having a specific complementarity to an epitope of the 2G12 antibody.

And yet another embodiment is a method of vaccinating and/or immunogenizing against HIV, comprising
administering to a subject a first composition comprising a glycoprotein, wherein N-glycans of said glycoprotein are predominantly high mannose glycans and a second composition comprising artificially selected having a specific complementarity to an epitope the 2G12 antibody, wherein the first and the second compositions are administered together or separately.

Still another embodiment is an antibody raised by vaccine or immunogenic composition of the present invention.

### BRIEF DESCRIPTION OF THE DRAWINGS

Figures 1A and 1B show antibody binding to kifunensine treated HIV-1_{IIIB} gp120.
Figures 2A and 2B show MALDI-MS of PNGase F-released glycans from target glycoprotein (RPTPmu) expressed in HEK 293T cells in the presence of 5 µM kifunensine.
Figure 3 shows mass spectrometric analysis of the characteristic structural fingerprint of normal Man9GlcNAc2 (top panel) and Man₉GlcNAc₂ derived from glycoproteins expressed in the presence of kifunensine (bottom panel).
Figure 4 shows antibody binding to 5 µM kifunensine treated CD48 and RPTPmu.
Figure 5 schematically illustrates structure of S. *cerivisiae* mannan indicating the α-linked mannose (circles) and the primary ligand of 2G12: Manα1-2Manα1-2Man (in box).
Figure 6 shows affinity of 2G12 for yeast cell surface over three rounds of selection.
Figures 7A and 7B present MALDI-TOF analysis of the PNGase-F released glycans for CD66a self glycoprotein expressed in untreated cells (top panel) and cells treated with kifunensine (low panel).
Figure 8 presents Enzyme-Linked Immunosorbent Assay (ELISA) data for 2G12 binding of the CD66a glycoprotein expressed in the presence of kifunensine.

### DETAILED DESCRIPTION

The present invention is directed to HIV vaccines, antibodies, and immunogenic compositions and methods of producing them, and, in particular, to carbohydrate HIV vaccines and immunogenic compositions and methods of producing them.

An HIV vaccine or immunogenic composition can be made by expressing a glycoprotein that the expressed glycoprotein has its glycosylation modified in such a way that the glycoprotein's affinity towards the 2G12 antibody increases compared of the same type of glycoprotein having unmodified, natural glycosylation.

The modification of glycosylation can be a result of expressing the glycoprotein in an expression system having altered glycosylation pathway or by expressing the glycoprotein in an expression system other than a natural expression system of the glycoprotein.

In the present context, altering a glycosylation pathway refers to either or both altering a genetic basis for glycan synthesis in the expression system and altering by exposing the expression system to chemical inhibitor(s) that disrupt/modify the activity of glycan processing enzymes.

In the present context, the term "modified glycosylation" means that glycans (oligosaccharides) of the glycoprotein expressed in the system with altered glycosylation pathway differ by at least one and preferably by more than one from glycan from the glycans that are naturally found on the glycoprotein.

The glycosylation of the glycoprotein can be modified in such a way that N-glycans on the glycoprotein are predominantly high mannose glycans. The term "predominantly" means that at least 50% , preferably at least 75%, more preferably at 90% and most preferably 95% of the N-glycans are high mannose glycans. High mannose glycans include glycans having at least one terminal Manα1,2Man linkage. Examples of such oligosaccharides are Man9GlcNAc2, Man8GlcNAc2, Man7GlcNAc2, Man6GlcNAc2 or their isomers. Preferably, N-glycans of the glycoprotein are predominantly Man9GlcNAc2 or its isomers. A content of N-glycan profile can be identified using known techniques. For example, N-glycans can released from the glycoprotein by PNGaseF and then analyzed by one or more high performance liquid chromatography, gel electrophoresis, mass spectrometry.

In some embodiments, the glycosylation pathway of the expression system can be altered by exposing the system chemical inhibitor(s) that disrupt/modify the activity of glycan processing enzymes. Such inhibitor can be glycosidase inhibitor, preferably α-mannosidase inhibitor. Table 1 presents an exemplary list of glycosidase inhibitors and their activity. Each glycosidase inhibitor can be used alone or in combination with other inhibitors.

**Table 1. Common inhibitors of the early glycosidases of the N-linked glycosylation pathway.**

| *Glycosidase Inhibitor* | *Glycosidase* |
|---|---|
| Australine | α 1-2 glucosidase I |
| Castonospermine | α 1-2 glucosidase I |
| Deoxynojirimycin | α 1-2 glucosidase II |
| 1,4-dideoxy-1,4-imini-D-mannitol (DIM) | Golgi α -mannosidase II |
| Deoxymannojirimycin | Golgi α 1-2 mannosidase I |
| Kifunensine | Golgi α 1-2 mannosidase I |
| 6-deoxy-DIM | Golgi α -mannosidase II |
| Mannostatin A | Golgi α -mannosidase II |
| Swainsonine | Golgi α -mannosidase II |
| D-mannonolactam amidrazone | α-mannosidases |
| Propylaminomannoamidine | α-mannosidase |

A particular concentration of glycosidase inhibitor can depend on the type of inhibitor, on the type of the glycoprotein being expressed. For example, the preferred mannosidase inhibitor, kifunensine, can be contacted with cells of the expression system in a concentration of no more than about 100 µM or no more than about 50 µM or no more than about 10 µM or no more than about 5 µM or no more than about 1 µM or nor more than about 0.5 µM.

The expression systems for the present invention can be high-yield mammalian expression systems such as human embryonic kidney 293T-E and S cells (HEK 293T), Chinese hamster ovary (CHO) and HepG2 cells.

In some embodiments, altering of glycosylation pathway of the expression system can be done by genetically manipulating glycosylation pathway. Thus, the expression system can mammalian expression system containing disrupted N-linked glycosylation to produce glycoproteins bearing oligomannose glycans can be, for example, deficient in alpha-mannosidase and/or GlcNAc-transferase I activity. The expression system can be also lectin resistant cell line including the cell lines deficient in alpha-mannosidase and/or GlcNAc-transferase I activity.

In some embodiments, the expression of glycoprotein can be carried out also in any expression system other than a natural expression system of the glycoprotein that modifies the glycosylation of the glycoprotein so it has an increased affinity to the 2G12 compared to the naturally found glycoprotein of the same type. Particularly contemplated expression systems include fungal/yeast cell lines, insect cell lines or mammalian cell lines with altered N-linked glycosylation genes for example the Lec-series mutants that are capable of expressing glycoproteins having high mannose structures. The yeast cell lines, for example, can be the mutant S. *cervesiae* Δ ochl, Δ mnnl (Nakanishi-Shindo, Y., Nakayama, K. I., Tanaka, A., Toda, Y. and Jigami, Y. (1993). Journal of Biological Chemistry 268: 26338-26345).

The glycosylation of the expressed glycoprotein is modified in such a way so that the affinity of the glycoprotein to the 2G12 antibody is increased compared to the glycoprotein of the same type with natural glycosylation. Conventional methods exist for determining an affinity of a glycoprotein to an antibody. One example of such method can be Enzyme-Linked Immunosorbent Assays (ELISA).

The glycoproteins that can be expressed according to the present invention include gp120 glycoprotein and "self'-glycoproteins. The glycoproteins can be obtained from any convenient source, for example, by standard recombinant techniques for production of glycoproteins.

### gp120

The glycosylation of naturally occurring gp120 is highly heterogeneous. The α1→2 linked structure, essential for 2G12 binding, is only present on the larger oligomannose glycans. Therefore, the two or three gp120 glycans that normally bind to 2G12 represent only a fraction of the total number of *N-*linked carbohydrates present on gp120 (up to 30 N-linked sites).

The degree of microheterogeneity of gp120 glycosylation, therefore, limits the number of binding sites for 2G12 and other similar anti-glycan antibodies. The more variable complex, hybrid and smaller oligomannose glycans are unable to support 2G12 binding. This limitation can be overcome by manipulation of the glycan processing pathway in order to restrict the glycan type(s) on gp120 to those which bind 2G12. This modification can be followed by an increased potential for this immunogen to elicit other antibodies with similar specificities to 2G12. Therefore, gp120 produced in the presence of kifunensine can act as an enhanced ligand not only for 2G12 but potentially for any other anti-mannose-cluster antibody, which may require mannose residues to be presented in other geometries.

### SELF PROTEINS

The immune response to gp120 is normally dominated by antibodies specific to the protein core. The *N-*linked glycans do not usually play a direct role in antibody recognition. To eliminate both the immune response to, and the immune modulation by, the protein moiety, 'self' proteins can be employed as scaffolds for 'non-self' oligomannose clusters. The expression of recombinant 'self' glycoproteins, in the presence of mannosidase inhibitors, or from a cell-line with a genetically manipulated glycosylation pathway, can provide a scaffold with oligomannose-type glycans, which mimic the 2G12 epitope. The advantage of this approach can be that the 2G12 epitope can be presented in an immunosilent, protein scaffold, with any antibody response directed only towards the oligomannose cluster.

The present invention also provides an HIV vaccine or immunogenic composition comprising mannans having specific complementarity to the 2G12 antibody. Mannans are polysaccharides containing mannose, preferably from yeast or bacterial cells. The mannans can be in the form of isolated mannans; whole yeast or bacterial cells, which may be killed cells or attenuated cells; or as mannans coupled to carrier molecule or protein. The mannans can be mannans for yeast or bacterial cells that a natural affinity to the 2G12 antibody. One example of such mannans can be mannan structures of *Candida albicans* that mimic the 2G12 epitope, i.e. have a natural specific complementarity to the 2G12 antibody.

The mannans can be also artificially or genetically selected mannans. Such mannans can be produced by iteratively selecting yeast or bacterial cells having a higher affinity to the 2G12 antibody. The starting pool of cells for this iterative process can comprise cells that exhibit some non-zero affinity or specificity. From the starting pool, a subset of cells can be selected that has a higher affinity to the 2G12 antibody than the rest of the cells. The cells of the subset can be then replicated and used as a starting pool for a subsequent iteration. Various criteria can be used for identifying a subset of cells having a higher affinity to the 2G12 antibody. For example, in a first iteration the cells that have a detectable affinity for the 2G12 antibody. In subsequent iterations, the selected cells can be cells representing The cells displaying a high affinity to the 2G12 antibody can selected out, using a fluorescence activated cell sorter (FACS), or by a direct enrichment using immobilized 2G12 for affinity separation.

One non-limiting example that can be used for a starting pool of cells are *S*. *cervisiae* cells. The 2G12 antibody can bind *S*. *cervisiae* mannans, thus, indicating a certain non-zero degree of antigenic mimicry between mannans and gp120 glycoprotein. The carbohydrate structure of *S*. *cerivisiae* cell wall shares common antigenic structures with the oligomannose glycans of gp120. However, naturally occurring *S*. *cervisiae* mannans do not induce sufficient humoral cross reactivity to gp120 when used as a immunogen.

The cells that can be used for the present invention can be also cells are deficient in one or more genes responsible for a mannan synthesis such as deficient in the mannosyl transferease gene product Mnn2p cells.

The vaccine or immunogenic composition can be administered for vaccinating and/or immunogenizing against HIV of mammals including humans against HIV. The vaccine or immunogenic composition can include mannans having a specific complementarity to the 2G12 antibody and/or a glycoprotein prepared according to described methods above. The glycoprotein can be included in the vaccine as isolated or purified glycoprotein without further modification of its glycosylation.

The vaccine or immunogenic composition can be administered by any convenient means. For example, the glycoprotein and/or mannans can administered as a part of pharmaceutically acceptable composition further contains any pharmaceutically acceptable carriers or by means of a delivery system such as a liposome or a controlled release pharmaceutical composition. The term "pharmaceutically acceptable" refers to molecules and compositions that are physiologically tolerable and do not typically produce an allergic or similar unwanted reaction such as gastric upset or dizziness when administered. Preferably, "pharmaceutically acceptable" means approved by a regulatory agency of the Federal or a state government or listed in the U.S. Pharmacopoeia or other generally recognized pharmacopoeia for use in animals, preferably humans. The term "carrier" refers to a diluent, adjuvant, excipient, or vehicle with which the compound is administered. Such pharmaceutical carriers can be sterile liquids, such as saline solutions, dextrose solutions, glycerol solutions, water and oils emulsions such as those made with oils of petroleum, animal, vegetable, or synthetic origin (peanut oil, soybean oil, mineral oil, or sesame oil). Water, saline solutions, dextrose solutions, and glycerol solutions are preferably employed as carriers, particularly for injectable solutions.

The vaccine or immunogenic composition can be administered by any standard technique compatible with the glucoproteins and/or mannans. Such techniques include parenteral, transdermal, and transmucosal, e.g., oral or nasal, administration. The following not-limiting examples further illustrate the present invention.

### Example 1. Production of gp120 in the presence of mannosidase inhibitors increases antigenecity.

The aim of the study is to generate modified gp120 molecules that can preferentially elicit broadly neutralising 2G12-like anti-HIV antibodies. An HIV-1*_{IIIB}* gp120 glycoprotein was produced in a Chinese hamster ovary (CHO) stable cell line using mannosidase inhibitors with the intention of modifying the glycoprotein to possess oligomannose epitope(s) of higher affinity for 2G12.

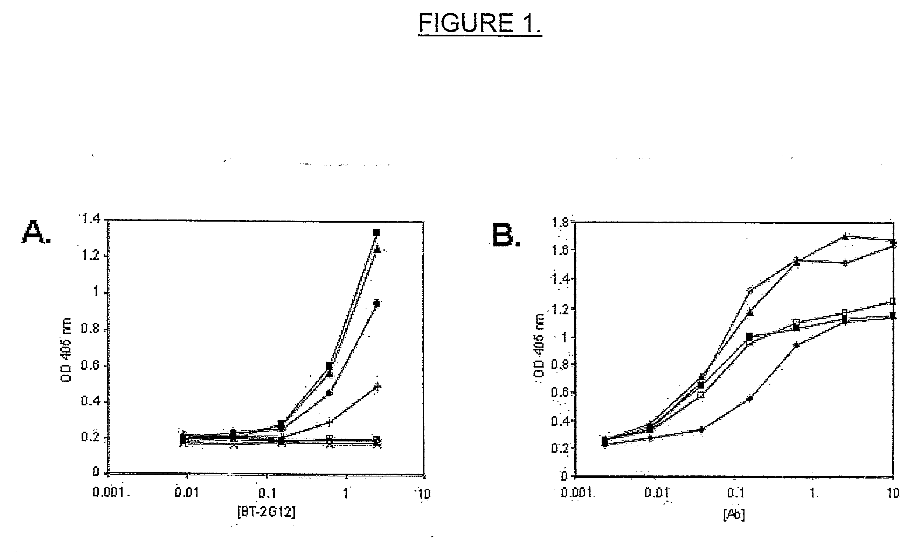

To investigate the role of mannosidase inhibition, by kifunensine, on the formation of the 2G12 epitope, Chinese Hamster Ovary (CHO) cells, transfected with EE6HCMVgp120GS, secreting recombinant HIV-1IIIB gp120, were cultured in CB2 DMEM Base culture medium supplemented with foetal calf serum (10%), penicillin (50 U/ml) and streptomycin (50 *¹*g/ml). All reagents were obtained from Gibco Ltd, Uxbridge, UK. High expression of gp120 was maintained by the addition of methionine sulphoximine (200 nM). Cells were grown in the presence and absence kifunensine (see Figure 1A, 1B).

Although both kifunensine and deoxymannojirimycin (DMJ, Table 1) both inhibit ER- and Golgi-resident class I α-mannosidases, kifunensine was selected as a mannosidase inhibitor in this study because it is able to effect mannosidase inhibition at 1000-fold lower concentrations than DMJ.

The production of CHO gp120 in the presence of the mannosidase inhibitor kifunensine resulted in a molecule that demonstrated higher binding to the monoclonal antibody 2G12 in Enzyme-Linked Immunosorbent Assays (ELISA).

Two 2G12 ELISA binding assays demonstrated that there was at least one additional 2G 12 binding site on each molecule of gp120, produced in the presence of kifunensine. Glycoproteins were immobilized on plastic protein-binding plates. For the first experiment (Figure 1A) 2G12 (5ug/ml) was coated onto plate, left overnight at 4oC. Plates were then blocked with Bovine Serum albumin (3% w/v) for one hour. Subsequently, supernatant from gp120IIIB expressing CHO cells (with or without kifunensine) was added for one hour, at room temperature. Plates were then washed 3 times in PBS. 2G12 (titration from 10yg/ml) was then added. After washing 2g12 binding was determined by phosphatase-conjugated anti-IgG secondary antibody, a final wash step and then phosphatase substrate measurement (p-nitrophenylphosphate, absorbance at 405nm).

The presence of additional binding site(s) as determined by b12 binding (Figure 1B) was performed by capturing gp120 with an anti-gp120 antibody (D7324) that does not compete with either 2G12 or b12 binding sites. Binding of gp120, and measurement of b12/2G12 was again determined by phosphatase-conjugated anti-IgG secondary antibody.

Figure 1 demonstrates antibody binding to kifunensine treated gp120 glycoprotein as detected via absorbance at 405nm from a phosphatase-conjugated anti-IgG secondary antibody for defined concentrations of 2G12, and control antibodies (ug/ml). Panel A of Figure 1 shows sandwich ELISA results demonstrating binding of more than one molecule of 2G12 to CHO gp120 produced in the presence of 0 µM kifunensine (open lozenge), 0.05 µM kifunensine (open triangle), 0.1 µM kifunensine (open square), 0.25 µM kifunensine (+), 0.5 µM kifunensine (filled triangle), 1 µM kifunensine (filled triangle) and 5 µM kifunensine (filled square). BSA (x) was used in these experiments as a negative control. Panel B of Figure 1 shows double antibody binding of 2G12 to kifunensine-treated CHO gp120. b12 binding to untreated gp120 (filled square), 2G12 binding to untreated gp120 (filled lozenge), b12 and 2G12 double antibody binding to gp120 (filled triangle); b12 binding (open square) and 2G12 binding (open lozenge) to gp120 produced in the presence of 5 µM kifunensine. The results from the sandwich ELISA assay show that with increasing kifunensine concentration, a higher proportion of gp120 molecules were able to simultaneously bind two or more 2G12 antibody molecules (Figure 1, panel A). A comparison of 2G12 binding to kifunensine treated gp120, with a double antibody ELISA of untreated gp120, (Figure 1, panel B) confirms the presence of two distinct epitopes for 2G12 on kifunensine treated gp120.

Conclusion: *N-*glycan analysis of kifunensine-treated glycoproteins indicate that the complex glycosylation is prevented leading to an oligomannoses glycoform, consistent with kifunensine's known inhibitory activity towards ER and Golgi resident mannosidases. As the result, the binding of 2G12 to a gp120 glycoprotein, expressed in the presence of kifunensine, is dramatically enhanced, with at least two 2G12 molecules able to bind to a single gp120 molecule.

### Example 2. Production of 'self' glycoproteins with antigenic cross-reactivity to HIV carbohydrates.

### a) CD 48 and RPTPmu

The aim of this study is to generate 'self' glycoproteins bearing oligomannose glycans which bind a 2G12 antibody and consequently display antigenic cross-reactivity with the HIV gp120. Two target glycoproteins, CD48 and Receptor Protein Tyrosine Phosphates mu (RPTPmu) were expressed in HEK293T cells in the presence of the mannosidase inhibitor kifunensine at 5 µM concentration. To verify that the mannosidase inhibitor was effective in producing glycoprotein containing oligomannose glycans, the glycans were released by digestion with protein *N-*glycanase F (PNGase F) and were then analysed by high-performance liquid chromatography (HPLC) and matrix assisted laser desorption/ionisation mass spectrometry (MALDI-MS).

Glycans were released from recombinant glyocprotiens by protein N-glycosidaseF digestion as described by Kurster et al (Anal. Biochem. 250(1)82-101) briefly: protein was separated by 10% SDS PAGE and the coomassie stained bands from the gel were cut out and frozen at - 20°C. The frozen gel pieces were then washed alternatively with acetonitrile and 20 mM Sodium bicarbonate buffer. This was followed by deglycosylation by enzymatic digestion overnight with PNGase F (EC 3.2.2.18, Roche Biochemicals) at 37°C in 20mM sodium bicarbonate buffer. The overnight reaction mix containing glycans was retained and any remaining glycans in the gel were extracted by sonication of the gel pieces with additional distilled water. Extracted glycans were finally purified for Mass spectrometry by passing through Micropure- EZ enzyme binding columns (Millipore, Bedford, MA, USA).

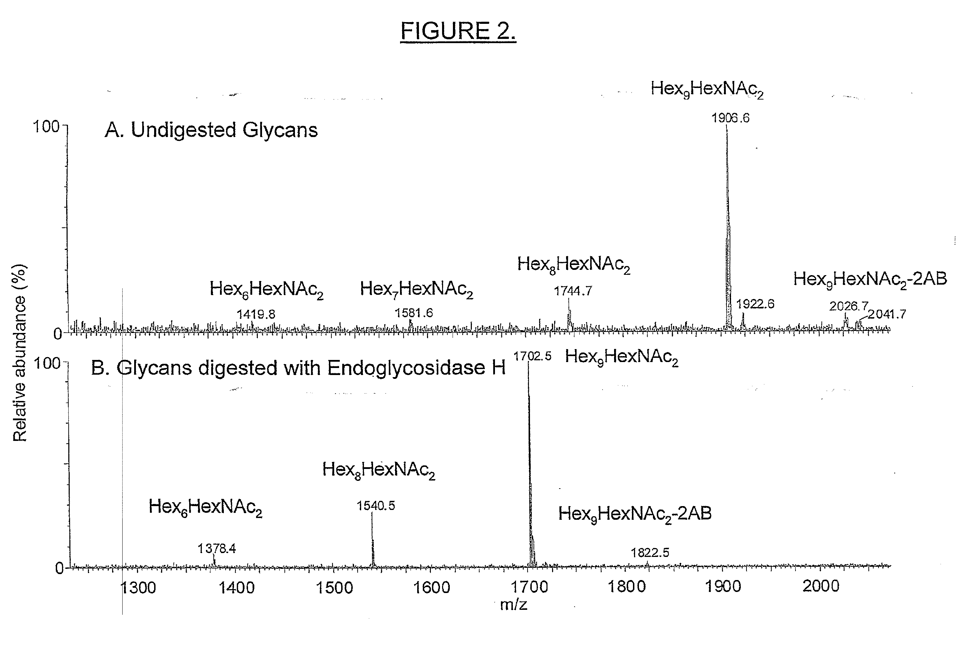

In addition, glycans were analysed following digestion with exo- and endo glycosidases. Figure 2 presents MALDI-MS of PNGase F-released glycans from target glycoprotein (RPTPmu) expressed in HEK 293T cells in the presence of 5 µM kifunensine. Data for undigested glycans and for glycans digested with endoglycosidase H are shown on Panels A and B of Figure 2 correspondingly.

Results of Figure 2 prove that the released glycan pool was entirely sensitive to endoglycosidase H digestion and alpha-mannosidase from Jack bean.

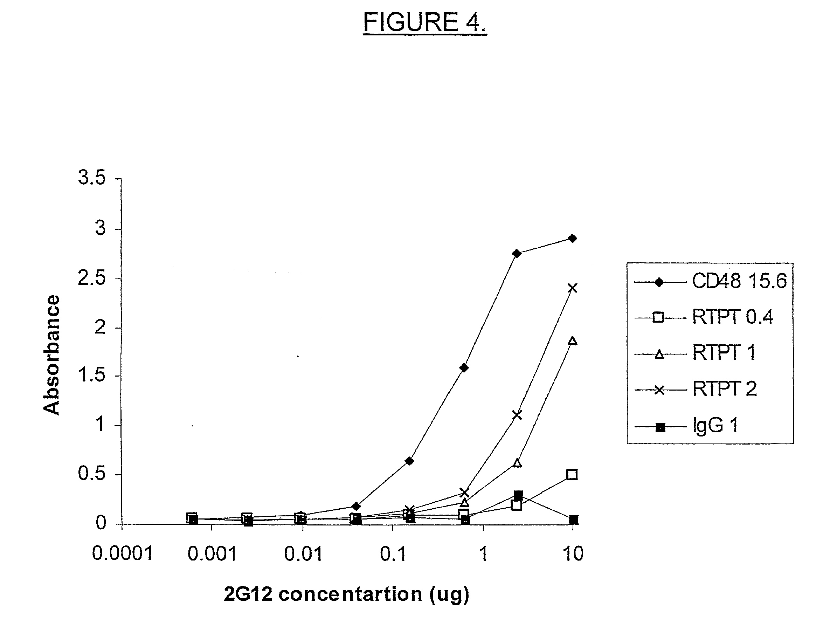

The resulting glycoproteins containing oligomannose-type *N-*linked glycans were tested for 2G12 binding by ELISA (Figure 4). Particularly, 15.6 µg of CD48, 2 µg, 1 µg and 0.4 µg of RPTPmu were plated and 1 µg of non-kifunensine treated IgG was plated as a negative control. Data of Figure 4 confirm that glycoproteins produced in the presence of mannosidase inhibitor can bind to 2G12 and, thus, are antigenic mimics of the HIV envelope glycoprotein, gp120.

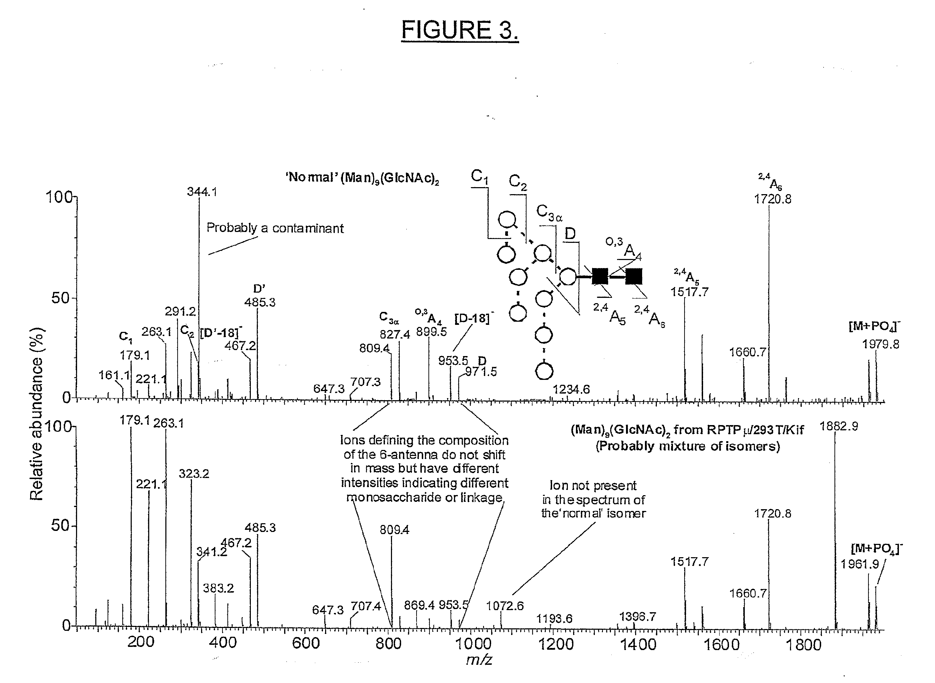

An antigenically significant feature of the glycans present on glycoproteins expressed in the presence of kifunensine is the generation of non-natural oligomannose isomers. In some expression systems, notably HEK 293T cells, the introduction of kifunensine can lead to the synthesis of Man₉GlcNAc₂ which, although of the same chemical composition as the natural isomer, can contain a different arrangement of the constituent monosaccharides. Figure 3 compares mass spectrometic analysis of the characteristic structural fingerprint of normal Man9GlcNAc2 (top panel) and

Man₉GlcNAc₂ derived from glycoproteins expressed in the presence of kifunensine (bottom panel) and confirms the existence of an antigenically novel Man₉GlcNAc₂ isomer.

Since the non-natural isomers present on glycoproteins derived from kifunensine treated cells are antigenically distinct from the Man₉GlcNAc₂ normally found on mammalian cells including HIV, one can expect that glycoproteins derived from kifunensine treated cells can exhibit an enhanced antigenic response when used as immunogens. Thus modification of the existing Man₉GlcNAc₂ structure may improve immungenicity above the clustering effect described.

As indicated in Figure 3, the oligomannose glycans from kifunensine treated HEK 293T cells, are of an antigenically 'non-self' isomer. Therefore, whilst retaining antigenic cross-reactivity to the native glycans of gp120, these novel glycans will exhibit an increased immunogenic capacity.

### b) CD66a

CD66a (CEACAM-1) self glycoprotein was expressed in HEK293T cells in the presence of the mannosidase inhibitor kifunensine at 50 µM concentration.

HEK293 cells transfected with rat CEACAM1 fused to human Fc were cultured in Dulbecco's Modified Eagle's Medium (DMEM) with 10% FCS, 100U/ml Penicillin, 100ug/ml streptomycin and 0.6 mg/ml G418. The Fc chimeric protein was allowed to accumulate for 10 days and purified using fast-flow protein A-Sepharose (Amersham Biosciences).

Western blotting analysis

The eluted protein was subjected to 10% SDS PAGE and electoblotted on to PVDF membrane (Immobillon-P, Millipore) using the tank-transfer apparatus (Bio-Rad, Hertfordshire, UK). Immunoblotting was done using 1:500 dilution of the monoclonal anti-CEACAM1 mouse monoclonal antibody Be9.2 (Kindly provided by Dr B.B.Singer) and the HRP conjugated anti-mouse antibody (1: 10,000 dilution). HRP-dependent luminescence was developed using the enhanced chemiluminescence

technique (ECL, Pierce, Northumberland, UK).

PNGase digestion and Glycan extraction

Purified Rat CEACAM1 protein was separated by 10% SDS PAGE and the
coomassie stained bands from the gel were cut out and frozen at - 20°C. The frozen gel pieces were then washed alternatively with acetonitrile and 20 mM Sodium bicarbonate buffer. This was followed by deglycosylation by enzymatic digestion overnight with PNGase F (EC 3.2.2.18, Roche Biochemicals) at 37°C in 20mM sodium bicarbonate buffer. The overnight reaction mix containing glycans was retained and any remaining glycans in the gel were extracted by sonication of the gel pieces with additional distilled water.

Extracted glycans were finally purified for Mass spectrometry by passing through Micropure- EZ enzyme binding columns (Millipore, Bedford, MA, USA).

To verify that the mannosidase inhibitor was effective in producing glycoprotein containing oligomannose glycans, the glycans were released by digestion with protein *N-*glycanase F (PNGase F) and were then analysed by matrix assisted laser desorption/ionization-time of flight- mass spectrometry (MALDI-TOF-MS).

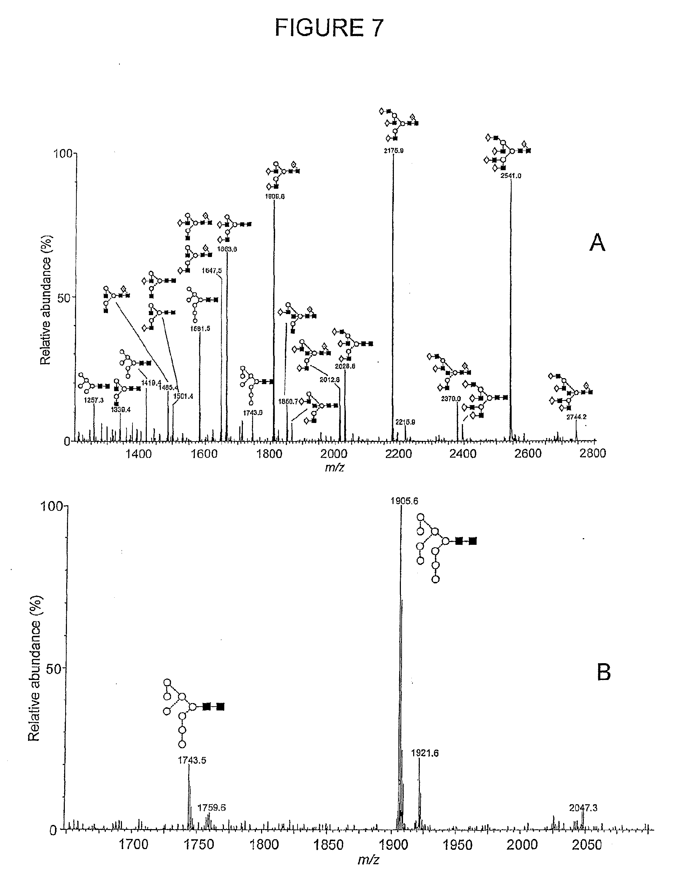

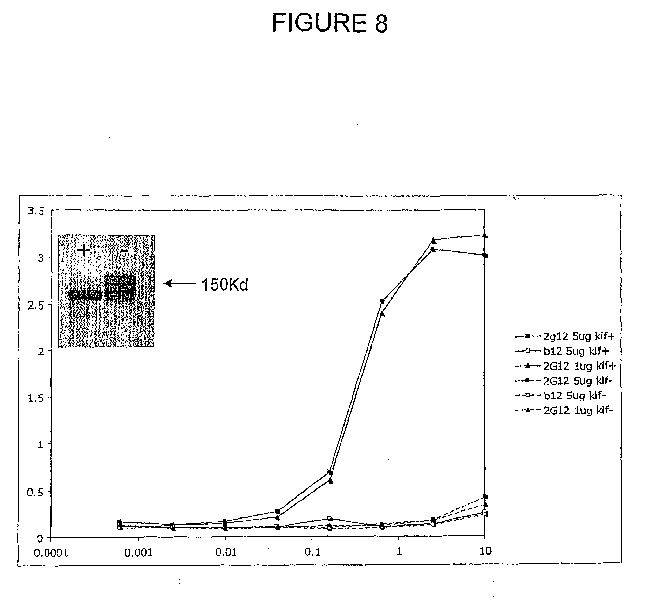

Figure 7 presents results of MALTI-TOF-MS analysis for glycans normally found on CD66a, i.e. for CD66a expressed in untreated cells, (top panel) and for glycans released from CD66a expressed in the presence of kifunensine (lower panel). The glycans normally found on CD66a form a diverse pool of complex N-linked carbohydrates, while glycans from CD66a expressed in the presence of kifunensine are oligomannose glycans, mostly GlcNAc2Man9. This reduction in glycan complexity correlates with the increase in CD66a affinity for the 2G12 antibody on Figure 8. The binding of 2G12 to immobilized CD66a was determined by ELISA. The effect of kifunensine treatment on glycan diversity on CD66a is also demonstrated by gel shift as a lower, more focused, apparent mass was observed for CD66a expressed in kifunensine presence (+) compared to normally found CD66a (-).

### Example 3. Binding of 2G12 to surface mannans of genetically selected yeasts.

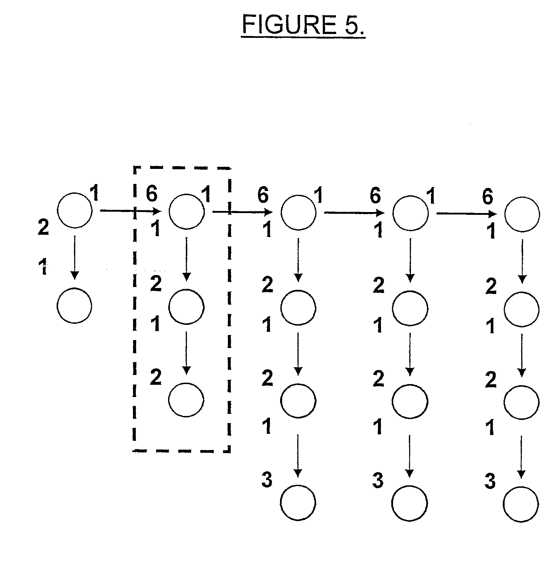

The strategy of selecting yeast mannans is to take an already immunogenic carbohydrate structure (*S. cerivisiae* mannan) and increase its antigenic similarity to gp120. For this study, both wild type S. *cerivisiae* (WT Mat-a B4741) and a strain deficient in the mannosyl transferease gene product Mnn2p (Δ*Mnn2* Mat-a B4741) were chosen. Many other pathogenic surfaces can share this structure, or can be evolved via artificial selection to do so. Particularly, the *ΔMnn2* mutant was selected because the terminal Manα1-3Man residues of branched mannan, whose addition is catalyzed by Mnn2p, would be expected to hinder 2G12 recognition (Figure 5). The binding of 2G12 to S. *cericisiae* mannans can be measured by fluorescence activated cell sorter (FACS). Cells, which display a detectable affinity for 2G12 are selected, and used to seed a daughter population. Repeated rounds of selection can drive the evolution of yeast mannans of higher affinity for 2G12.

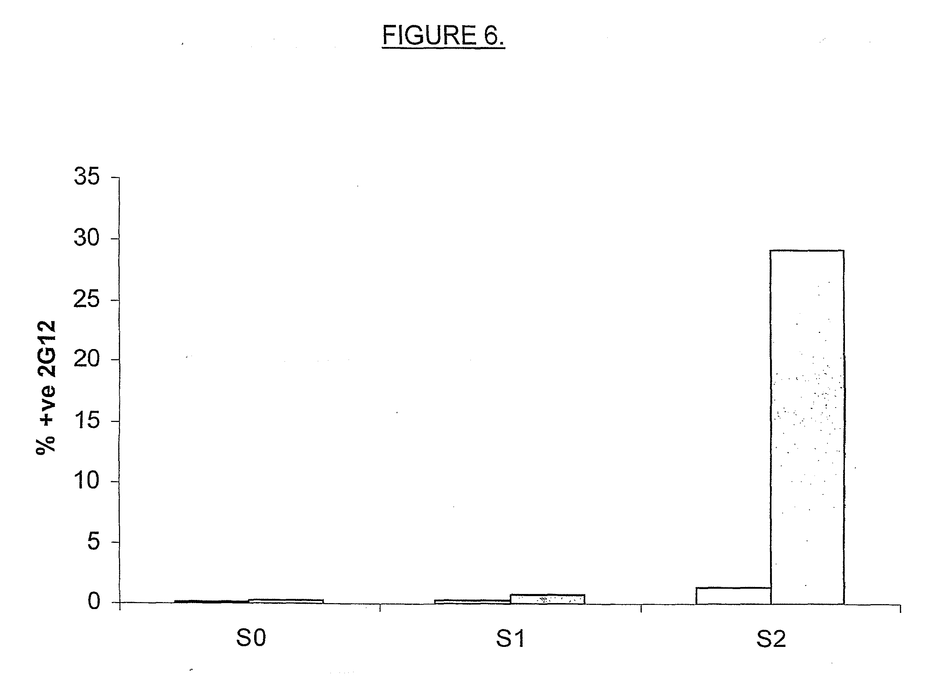

Figure 6 demonstrates affinity of 2G12 for yeast cell surface over three rounds of selection. The value on y-axis indicates the fraction of yeast cells which bind to 2G12 with a higher affinity than did 99.5% of the initial WT population. The evolution of WT (clear bars) and *ΔMnn2* (shaded bars) populations are indicated on Figure 6. Data of Figure 6 indicate that the selection of yeasts, according to their ability to bind 2G12, can lead to a heritable increase in 2G12 affinity for the cell surface. The *ΔMnn2* strain, as anticipated, is better able to support such an adaptation to the selection criteria than WT. Additional rounds of selection and replication can continue to alter the mannan structure and, thus, increase their antigenic mimicry of the 2G12 epitope. Mannan structures thus produced can be used for immunization studies, both in isolation, and as protein conjugates.

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention.

All of the publications, patent applications and patents cited in this specification are incorporated herein by reference in their entirety.

## Claims

1. An HIV vaccine or immunogenic composition, comprising mannans having a specific complementarity to an epitope of the 2G12 antibody.

2. The vaccine or composition of claim 1, wherein said mannans are mannans of yeast or bacterial cells.

3. The vaccine of claim 1, wherein said mannans are artificially selected mannans.

4. A composition comprising artificially selected mannans having a specific complementarity to an epitope of the 2G12 antibody for use in the vaccination and/or immunogenization against HIV.

5. A method of producing an HIV vaccine or immunogenic composition comprising performing at least one time an iteration comprising:
(i) selecting from a first pool of cells a subpool of cells, wherein the cells of the subpool have a higher affinity to the 2G12 antibody than the cells of the first pool; and
(ii) replicating the cells of the subpool to produce a second pool of cells;
wherein the vaccine or composition comprises the cells of the second pool from a last iteration.

6. The method of claim 5, performing said iteration two or more times,
wherein the second pool of cells of a non-last iteration is the first pool of cells of an iteration immediately following the non-last iteration.

7. The method of claim 5, wherein the cells of the first pool are yeast cells.

8. The method of claim 7, wherein the yeast cells are *Candida albicans* cells or S. *cerivisae* cells.

9. The method of claim 7, wherein said yeast cells are deficient in one or more genes responsible for a mannan synthesis.

10. The method of claim 5, wherein said selecting is carried out by a fluorescent activated cell sorter or by a direct enrichment using immobilized 2G12 antibody for affinity separation.

11. An HIV vaccine or immunogenic composition produced by the method of claim 5.

12. An HIV vaccine or immunogenic composition comprising:
(i) artificially selected mannans having a specific complementarity to an epitope of the 2G12 antibody; and
(ii) a glycoprotein, wherein N-glycans of said glycoprotein are predominantly high mannose glycans.

13. A combination of:
- a first composition comprising a glycoprotein, wherein N-glycans of said glycoprotein are predominantly high mannose glycans, and
- a second composition comprising artificially selected mannans having a specific complementarity to an epitope the 2G12 antibody,
for use in the vaccination and/or immunogenization against HIV,
wherein the first and the second compositions are administered together or separately.
